Europäisches Patentamt

European Patent Office

Office européen des brevets

(11) Veröffentlichungsnummer: **0 256 353**

**A2**

# (12) EUROPÄISCHE PATENTANMELDUNG

(21) Anmeldenummer: 87110746.2

(22) Anmeldetag: 24.07.87

(51) Int. Cl.⁴: **A61K 35/78**

(30) Priorität: 09.08.86 DE 3627010

(43) Veröffentlichungstag der Anmeldung:
24.02.88 Patentblatt 88/08

(84) Benannte Vertragsstaaten:
AT BE CH DE ES FR GB IT LI NL SE

(71) Anmelder: **Badmajew, Vladimir, Dr.**
**1440/6 Forest Hill Road**
**Staten Island New York 10314(US)**

(72) Erfinder: **Badmajew, Vladimir, Dr.**
**1440/6 Forest Hill Road**
**Staten Island New York 10314(US)**

(74) Vertreter: **Bauer, Wulf, Dr.**
**Wolfgang-Müller-Strasse 12**
**D-5000 Köln 51 (Marienburg)(DE)**

(54) Pharmazeutikum zur Therapie einer schlechten Absorption von Nahrungsmitteln und von Oral einzunehmenden Medikamenten.

(57) Das Pharmazeutikum mit den vier pflanzlichen Bestandteilen: Neun Gewichtsteile Zingiberis rhizoma, fünf Gewichtsteile Myrobalan, acht Gewichtsteile Rhei chenensis radix und fünf Gewichtsteile Inulae helenii radix sowie mit dem mineralischen Bestandteil fünf Gewichtsteile Natrium sulfuricum siccum ist als Pharmazeutikum zur Therapie einer - schlechten Absorption von Nahrungsmitteln und von (anderen) oral einzunehmenden Medikamenten bestimmt. Es wird vorzugsweise in homöopathischen Dosierungen verabreicht und eignet sich zur Prophylaxe gegen Dickdarmkrebs.

## Pharmazeutikum zur Therapie einer schlechten Absorption von Nahrungsmitteln und von oral einzunehmenden Medikamenten

Die Erfinding bezieht sich auf ein Medikament zur Therapie einer schlechten Absorption von Nahrungsmitteln und von (anderen) oral einzunehmenden Medikamenten.

Der Zustand des Verdauungstraktes ist für die Aufnahme von Nahrungsmitteln und von oral einzunehmenden Medikamenten von entscheidender Bedeutung. Schlechte Ernährungsgewohnheiten oder falsche Ernährung können den Zustand des Verdauungstraktes so beeinflussen, daß Nahrungsmittel schlecht oder unzureichend absorbiert werden und oral eingenommene Medikamente nicht so aufgenommen werden, es für ihre Wirksamkeit notwendig ist. Zu schlechten Ernährungsgewohnheiten sind beispielsweise fettreiche und proteinreiche Mahlzeiten zu zählen, die einen geringen Anteil an Ballaststoffen haben.

Der Zustand des Verdauungstraktes wird insbesondere durch den Zustand der Schleimstoffe, die den gesamten Verdauungstrakt auskleiden und als natürliche Gleitmittel, als Epithelschutz usw. dienen, bestimmt. Diese Schleimstoffe sind uneinheitliche Sekrete der Schleimdrüsen bzw. Schleimzellen und treten als Speichel, Magensaft, Darmsaft usw. auf.

Der Erfindung liegt die Aufgabe zugrunde, ein Pharmazeutikum zur Therapie der schlechten Absorption von Nahrungsmitteln und von (anderen) oral einzunehmenden Medikamenten anzugeben. Diese Aufgabe wird gelöst durch ein Medikament mit den vier pflanzlichen Bestandteilen, nämlich neun Gewichtsteile Zingiberis rhizoma, fünf Gewichtsteile Myrobalan, acht Gewichtsteile Rhei chinensis radix und fünf Gewichtsteile Inulae helenii radix sowie mit einem mineralischen Bestandteil in Form von fünf Gewichtsteilen Natrium sulfuricum siccum.

Dieses Pharmazeutikum regt die Produktion von Schleimstoffen durch die gastro-intestinalen Schleimdrüsen an, verbessert die Peristaltik des Verdauungstraktes und hat auf Grund des großen Anteils an pflanzlichen Bestandteilen einen hohen Ballastwert, der Ballast erhöht das Stuhlvolumen. Durch die Einnahme des erfindungsgemäßen Pharmazeutikums wird die Funktion des Verdauungstraktes reguliert, so daß die Absorption von Nahrungsmitteln verbessert wird. Insbesondere wird nach einer etwa einwöchigen bis zehntägigen Einnahme des erfindungsgemäßen Pharmazeutikums der Verdauungstrakt in die Lage versetzt, andere oral einzunehmende Medikamente so aufzunehmen, daß sie in ihrer beabsichtigten Dosis auch tatsächlich zur Wirkung gelangen.

Von den fünf Bestandteilen des erfindungsgemäßen Pharmazeutikums sind Rhei chinensis radix und Natrium sulfuricum (Glaubersalz) in ihrer laxativen Wirkung bekannt. Die Dosierung dieser Bestandteile soll nun so erfolgen, daß die laxative Wirkung noch nicht oder zumindest noch nicht merklich eintritt. Insbesondere soll verhindert werden, daß durch die beiden laxativ wirkenden Bestandteile der Verdauungstrakt leer geräumt wird und sich die Flora und die Schleimstoffe erst wieder aufbauen müssen. Aufgrund der für eine laxative Wirkung nicht ausreichenden Dosierung wird die Funktion des Verdauungstraktes angeregt und eine verbesserte Sekretabsonderung erzielt. Diese Wirkungen sollen aber nur die normale Verdauung unterstützen und sicherstellen, daß der Verdauungstrakt intakt ist, also gut und ordnungsgemäß funtioniert.

Das erfindungsgemäße Pharmazeutikum ist somit einerseits ein echtes Medikament, da es positiv und heilend auf den Zustand des Verdauungstraktes einwirkt. Es stellt dadurch sicher, daß Nahrungsmittel auch tatsächlich in ihrem vollen Umfang aufgenommen werden und vermeidet, daß durch eine zu Anfang eines Mahls erfolgende starke Überlastung des Verdauungstraktes beispielsweise durch Schokolade, kurzzeitig später aufgenommene, wertvolle Nahrungsmittel nicht einfach den Verdauungs trakt passieren, sondern auch ihrerseits aufgeschlossen und absorbiert werden. Andererseits ist das erfindungsgemäße Pharmazeutikum aber auch als eine Art Promedikament zu verstehen, das erst den Weg für ein anderes, oral einzunehmendes Medikament ebnet und durch seinen positiven Effekt auf den Verdauungstrakt sicherstellt, daß das (eigentliche) Medikament auch tatsächlich so vom Verdauungstrakt aufgenommen wird, wie es für die Heilbehandlung notwendig ist.

Die beiden genannten laxativen Bestandteile können noch durch weitere laxative Bestandteile ergänzt werden, beispielsweise Aloe vera, Cascara sagrada, Rhamnus frangula und Rheum plamatum. Aloe ist ein langsam wirkendes Laxativ, die Wirkung stellt sich typischerweise achtzehn bis zwanzig Stunden nach Einnahme ein. Aloe ist weitgehend unwirksam gegen chronische Verstopfung. Cascara andererseits ist für die Behandlung chronischer Verstopfungen geeignet, es ist ebenfalls ein langsam wirkendes Laxativ. Rhamnus hat eine der Cascara ähnlich Wirkung, beide Pflanzen gehören zur Familie der Rhamnaceae. Rheum ist im Gegensatz zu den drei anderen pflanzlichen Laxativen ein rasch wirkendes (innerhalb weniger Stunden wirkendes) Abführmittel.

Rhei chinensis radix und Natrium sulfuricum bilden, vorzugsweise ergänzt durch zumindest eine der vier weiteren, laxativ wirkenden Heilpflanzen, insbesondere aber in Kombination mit allen diesen zusätzlichen vier Heilpflanzen, die erste therapeutische Gruppe im erfindungsgemäßen Pharmazeutikeum. Eine zweite therapeutische Gruppe wird durch Myrobalan gebildet und kann ergänzt werden durch Marsdenia condurango, Piper longum, und/oder Strychnos nux-vomica. Diese zweite therapeutische Gruppe unterstützt die laxative Wirkung, indem sie die Peristaltik anregt und den Wasserhaushalt des Darmbereiches regelt. Strychnos und Condurango stimulieren die Darmbewegungen. Diese Wirkung hat auch Piper, das insbesondere die rektale Schleimhaut stimuliert. Sein Bestandteil Piperin hat eine zentrale analeptische Aktitvät. Sodiumsulfat erhöht den Wasseranteil im Stuhlgang.

Die laxative Wirkung der ersten und zweiten therapeutischen Gruppen ist nicht der einzinge oder entscheidende Effekt des erfindungsgemäßen Phar mazeutikums. Es ist jedoch ein wesentlicher Mechanismus für die sehr große Wirkungsbreite im Magen-Darmbereich. Zusätzlich zu ihrer laxativen Wirkung verstärken die meisten Pflanzen der ersten und zweiten therapeutischen Gruppe (Aloe, Condurango, Myrobalan, Piper, Rheum und Strychnos) die gastrointestinale Sekretion, einschließlich der sekretorischen Aktivität der Leber und Bauchspeicheldrüse.

Die dritte therapeutische Gruppe umfaßt Pflanzen und Mineralien, die die Verdauung, Absorption und den Aufschluß der anderen Bestandteile fördern, insbesondere durch Einwirkung auf die gastrointestinale Sekretion. Die dritte therapeutische Gruppe beseitigt auch Nebeneffekte der laxativen Bestandteile und reguliert die Darmflora. Neben Inulae und Zingiberis können (zusätzlich) diese dritte Gruppe bilden Aloe vera, Aluminiumsilikat (Kaolin), Jateorhiza palmata (Caumba), Marsdenia condurango, Piper longum und/oder Rheum palmatum.

Einige der genannten Bestandteile tauchen in mehr als einer therapeutischen Gruppe auf. Dieser zusätzliche und ergänzende Mechanismus ist entscheidend und wichtig, weil die Dosis aller individuellen Bestandteile unterhalb der therapeutischen Dosis liegt und ein einzelner Bestandteil für· sich allein praktisch keine Wirkung hat.

In Weiterbildung der Erfindung wird vorgeschlagen, daß die Verabreichung der erfindungsgemäßen pharmazeutischen Mischung in homöopathischen Dosen erfolgt. Eine homöopathische Dosierung wird bei den pflanzlichen Bestandteilen schon dadurch erzielt, daß in diesen die eigentlichen Wirkstoffe nur in sehr geringen Mengen vorhanden sind und der größte Anteil der vier pflanzlichen Bestandteile Ballastoffe sind.

Weiterhin wird vorgeschlagen, daß die pflanzlichen Bestandteile getrocknet, zu einem sehr feinen Pulver zerkleinert und dann gemeinsam mit dem Glaubersalz vermischt und zu Tabletten verpreßt werden. Dabei liegt auch das Glaubersalz in Form eines sehr feinen Pulvers vor. Aufgrund dieser sehr feinen Verteilung aller fünf Bestandteile wird eine gute und großflächige Verteilung des Pharmazeutikums an den Innenwänden des Verdauungstraktes ermöglicht, hohe lokale Konzentrationen werden vermieden. Da das Pharmazeutikum lediglich zu Tabletten verpreßt wird, die sich sehr leicht auflösen können, wird eine rasche und großflächige Verteilung in Form einer Auskleidung der Verdauungstraktswände erreicht.

Als besonders vorteilhaft hat sich erwiesen, das in Tablettenform verabreichte Pharmazeutikum nicht mit irgendeinem Überzug zu umhüllen. Dadurch wird sein leicht bitterer Geschmack im Mund wahrgenommen, was zu einer Anregung der Speichelsekretion führt und somit zur therapeutischen Wirkung des Pharmazeutikums beiträgt.

Ein Vorteil des Pharmazeutikums aus den oben genannten vier pflanzlichen Bestandteilen und dem einen mineralischen Bestandteil wird in der Verbesserung der Absorption von Nahrungsmitteln und von (anderen) oral einzunehmenden Medikamenten gesehen, in dieser Anwendung liegt einer der Hauptgedanken der Erfindung.

Darüberhinaus hat sich die Anwendung des erfindungsgemäßen Pharmazeutikums als positiv zur Vorbeugung gegen Dickdarmkrebs erwiesen. Diese überraschende Schutzfuntion wird darauf zurückgeführt, daß bei intaktem Verdauungstrakt krebserzeugende Substanzen schlechter zur Wirkung kommen bzw. besser abgewehrt werden.

Das erfindungsgemäße Pharmazeutikum hat einen antiseptischen Effekt auf die Darmflora und reguliert die Fäulnißbildung, übermäßte Fäulnißbildung wird unterdrückt.

Beispiel I:

In einer Tablette des erfindungsgemäßen Pharmazeutikums sind enthalten

    30 mg Zingiberis rhizoma,
    I7,5 mg Myrobalanum,
    25 mg Rhei chinensis radix
    I7,5 mg Inulae helenii radix
    I7,5 mg Natrium sulfuricum siccum

Höher verdünnte Darreichungsformen unter Beibehaltung der gewichtsmäßigen Abstufung nach Patentanspruch I sind ausdrücklich nicht ausgeschlossen. Die Dosierung der beiden laxativ wirkenden Bestandteile Rheii chinensis radix und Natrium sulfuricum soll insbesondere einhundertmal geringer sein als die therapeutische Dosis, wie sie für die laxative Wirkung dieser beiden Bestandteile allgemein empfohlen wird.

Rhizoma zingiberis ist der Wurzelstock des indischen Ingwer. Myrobalan ist das griechische Wort für Salbeneiche, verwendet wird die Frucht der Langfadengewächsart Terminalia chebula des tropischen Asiens und der malaiischen Inseln. Terminalia chebula hat einen beträchtlichen Anteil an Tannin, der zwischen 20 und 40 Prozent liegen kann. Dieser hohe Trannin-Gehalt erklärt in einem gewissen Maße die breite Anwendbarkeit dieses Pflanzenstoffes im gastrointestinalen Bereich. Tannine sind adstringierend, sie binden sich mit und fällen Proteine aus und bilden eine protektive Schicht, die gegenüber proteolytischen Enzymen resistent ist.

Rhei chinensis radix ist der geschälte Wurzelstock von Rhabarber aus der Familie der Polygonaceae. Die Wirkung von Rhei chinensis radix wird bedingt durch Antrachinonglykoside, denen allgemein abführende Wirkung im Dickdarm zugeschrieben wird. Auch Rhei chinensis radix hat einen beträchtlichen Tannin-Gehalt, zu dessen Wirkungen bereits oben Stellung genommen wurde.

Inulae helenii radix hat den deutschen Namen wahrer Alant. Inulae helenii enthält Helenin, das aus Alantolactonen und Isoalantolactonen besteht. Es ist ein sehr wirksames Antiseptikum sowohl in vitro als auch in vivo. Eine 1 : 10 000 Verdünnung von Helenin ist bakterizid in vitro.

Im folgenden wird auf die beobachtete prophylaktische Wirkung des erfindungsgemäßen Pharmazeutikums gegen Dickdarmkrebs eingegangen: Die Auswirkung der Ernährung auf Krebserkrankungen ist von hohem Interesse, da Ernährungsfaktoren nach Tabak allgemein als zweitwichtigste Faktoren für Krebs gelten. Ballastoffe wurden sowohl epidemiologisch als auch experimentell in ihrer Wirkung zur Krebsprophylaxe untersucht. Die epidemio logischen Untersuchungen deuten einen geringeren Dickdarmkrebsbefall für untersuchte Personen an, die Nahrung mit hohem Ballaststoffanteil zu sich nahmen. Die experimentellen Studien weisen auf einen Zusammenhang zwischen Ballaststoffen und Fett bei der Auslösung von Dickdarmkrebs hin. Der Grund, warum eine ballaststoffreiche Ernährung dazu helfen kann, gegen Dickdarmkrebs vorzubeugen, ist noch nicht ausreichend verstanden. Durch die Ballaststoffe wird das Volumen der im Verdauugstrakt befindlichen Stoffe erhöht, ebenso wird das Volumen des Stuhls vergrößert, was zu der Vermutung führt, daß eine herabgesetzte Konzentration von Karzinogenen und ihre verringerte Einwirkungszeit die Hauptursachen für den antikarzinogenen Effekt von Ballaststoffen sind.

Insgesamt wird davon ausgegangen, daß die Bestandteile des erfindungsgemäßen Pharmazeutikums den Kontakt von Karzinogenen mit dem intestinalen Epithel verringern. Das erfindungsgemäße Pharmazeutikum hat hierdurch einen prophylaktischen Effekt gegen chemisch induzierten Dickdarmkrebs.

Beispiel 2:

57 mg Zingiberis rhizoma,
32 mg Myrobalanum,
53 mg Rhei chinesis radix
30 - 50 mg Inulae helenii radix
32 mg Natrium sulfuricum siccum
40 mg Aloe vera
25 mg Aluminiumsilikat
18 mg Jateorhiza
18 mg Marsdenia condurango
4,5 mg Piper longum
50 mg Cascara sagradae
50 mg Rhamnus frangulae

**Ansprüche**

I. Pharmazeutikum zur Therapie einer schlechten Absorption von Nahrungsmitteln und von (anderen) oral einzunehmenden Medikamenten mit den vier pflanzlichen Bestandteilen: Neun Gewichtsteile Zingiberis rhizoma, fünf Gewichtsteile Myrobalan, acht Gewichtsteile Rhei chinensis radix und fünf Gewichtsteile Inulae helenium radix sowie fünf Gewichtsteile Natrium sulfuricum siccum als mineralischer Bestandteil.

2. Pharmazeutikum nach Anspruch I, dadurch gekennzeichnet, daß die Verabreichung in homöopathischer Dosierung erfolgt.

3. Pharmazeutikum nach Anspruch I oder 2, dadurch gekennzeichnet, daß die Verabreichung der als laxativ wirkenden Bestandteile Rhei chinensis radix und Natrium sulfuricum in einer Verdünnung erfolgt, die geringer und vorzugsweise einhundertmal kleiner ist als die für die laxative Wirkung verwendete Dosis.

4. Pharmazeutikum nach einem der Ansprüche I bis 3, dadurch gekennzeichnet, daß die pflanzlichen Bestandteile getrocknet, zu einem sehr feinen Pulver zerkleinert, gemeinsam mit dem mineralischen Bestandteil gemischt und zu Tabletten verpreßt sind.

5. Pharmazeutikum nach einem der Ansprüche I bis 4, dadurch gekennzeichnet, daß die Oberfläche der gepreßten Tabletten durch die Bestandteile selbst gebildet wird, insbesondere daß die Tabletten nicht durch einen Überzug umhüllt sind.

6. Pharmazeutikum nach einem der Ansprüche I bis 5, dadurch gekennzeichnet, daß eine Tablette 0,03 g Zingiberis rhizoma, 0,0175 g Myrobalan, 0,025 g Rhei chinensis radix, 0,0175 g Insulae helenii radix, 0,0175 g Natrium sulfuricum siccum enthält.

7. Verwendung eines Pharmazeutikums mit neun Gewichtsteilen Zingiberis rhizoma, fünf Gewichtsteilen Myrobalan, acht Gewichtsteilen rhei chinensis radix und fünf Gewichtsteilen inulae helenii radix an pflanzlichen Bestandteilen sowie fünf Gewichtsteilen Natrium sulfuricum siccum als mineralischen Bestandteil zur Verbesserung der Absorption von Nahrungsmitteln und von (anderen) oral einzunehmenden Medikamenten bei Verabreichung in homöopathischen Dosen und vorzugsweise in einer Dosierung der laxativen Bestandteile, die unter der für eine laxative Wirkung liegt.

8. Verwendung eines Pharmazeutikums mit vier Gewichtsteilen Zingiberis rhizoma, fünf Gewichtsteilen Myrobalan, acht Gewichtsteilen rhei chinensis radix, fünf Gewichtsteilen inulae helenii radix als pflanzlichen Bestandteilen unf fünf Gewichtsteilen Natrium sulfuricum siccum als mineralischem Bestandteil zur Prophylaxe gegen Dickdarmkrebs, vorzugsweise bei Verabreichung in homöopathischen Dosen.